# EUROPÄISCHE PATENTANMELDUNG

(11) **EP 3 150 264 A1**
(43) Veröffentlichungstag der Anmeldung: **05.04.2017**
(21) Anmeldenummer: 15187795.8
(22) Anmeldetag: 30.09.2015
(51) Int. Cl.: B01D 11/02, B03D 1/10, C07D 311/00

(54) **VAKUUMDESTILLATION ZUR ANREICHERUNG VON CBD**

(71) Anmelder: Bionorica Ethics GmbH, 92318 Neumarkt (DE)
(72) Erfinder: RUTZ, Andreas, 91741 Dornhausen (DE); ENGLERT, Michael, 90478 Nürnberg (DE)
(74) Vertreter: Simandi, Claus

(57) **Zusammenfassung**

Die vorliegende Erfindung betrifft ein Verfahren zur Herstellung, Gewinnung und Anreicherung von Cannabidiol (CBD) und Verwendung. Die Erfindung betrifft ferner einen Cannabidiol-Extrakt und die Verwendung eines erhaltenen Cannabidiol-Extraktes in der Pharmazie und Kosmetik.

## Beschreibung

Die vorliegende Erfindung betrifft ein Verfahren zur Herstellung, Gewinnung und Anreicherung von Cannabidiol (CBD) und Verwendung. Die Erfindung betrifft ferner einen Cannabidiol-Extrakt und die Verwendung eines erhaltenen Cannabidiol-Extraktes in der Pharmazie und Kosmetik.

Cannabis (Hanf) gehört zusammen mit der Gattung Humulus (Hopfen) der Familie der Cannabisaceae an, wobei jedoch Humulus keine Cannabinoide enthält. Innerhalb der Gattung Cannabis erfolgt eine botanische und chemotaxonomische Differenzierung und zwar in den Arten Cannabis sativa Linnaeus, Cannabis indica LAM und Cannabis ruderalis oder in die "Sammelart" Cannabis sativa L., bestehend aus den Unterarten Cannabis sativa ssp. sativa und ssp. indica. Darüber hinaus wird Cannabis in einen Drogen- und Faserhanf unterschieden, wobei die Unterscheidung aufgrund des mengenmäßigen Verhältnisses der Hauptcannabinoide Cannabidiol (CBD) und Δ⁹-Tetrahydrocannabinol (Δ⁹-THC) erfolgt (INN: Dronabinol). Faserhanf (auch: Nutzhanf, industrial hemp) wird hauptsächlich zur industriellen Fasergewinnung herangezogen und darf maximal ein Δ⁹-THC-Gehalt von 0,2 % aufweisen (z.B. Deutschland u.a.), während der Drogentyp ein Δ⁹-THC-Gehalt von ca. 5-15 % aufweisen kann (Marihuana, Haschisch). Cannabis sativa L. enthält über 400 verschiedene Inhaltsstoffe, davon gehören mehr als 60 Verbindungen der Klasse der Cannabinoide an. Die wichtigsten Cannabinoide sind im Folgenden dargestellt:
*Cannabigerol-artige (CBG) :* Cannabigerol ((E)-CBG-C₅), Cannabigerol Monomethylether ((E)-CBGM-C₅ A), Cannabinerolsäure A ((Z)-CBGA-C₅ A), Cannabigerovarin ((E)-CBGV-C₃), Cannabigerolsäure A ((E)-CBGA-C₅ A), Cannabigerolsäure A Monomethylether ((E)-CBGAM-C₅ A), Cannabigerovarinsäure A ((E)-CBGVA-C₃ A) ;
*Cannabichromen-artige (CBC) :* Cannabichromen (CBC-C₅), Cannabichromensäure A (CBCA-C₅ A), Cannabichromevarin (CBCV-C₃), Cannabichromevarinsäure A (CBCVA-C3 A);
*Cannabidiol-artige (CBD):* Cannabidiol (CBD-C₅), Cannabidiol Monomethylether (CBDM-C₅), Cannabidiol-C4 (CBD-C₄), Cannabidivarin (CBDV-C₃), Cannabidiorcol (CBD-C₁), Cannabidiolsäure (CBDA-C₅), Cannabidivarinsäure (CBDVA-C₃);
*Cannabinodiol-artige (CBND) :* Cannabinodiol (CBND-C₅), Cannabinodivarin (CBND-C₃);
*Tetrahydrocannabinol-artige (THC):* Δ9-Tetrahydrocannabinol (Δ9-THC-C₅), Δ9-Tetrahydrocannabinol-C4 (Δ9-THC-C₄), Δ9-Tetrahydrocannabivarin (Δ9-THCV-C₃), Δ9-Tetrahydrocannabiorcol (Δ9-THCO-C₁), Δ9-Tetrahydrocannabinolsäure (Δ9-THCA-C₅ A), Δ9-Tetrahydrocannabinolsäure B (Δ9-THCA-C₅ B), Δ9-Tetrahydrocannabinolsäure-C4 (Δ9-THCA-C₄ A und/oder B), Δ9-Tetrahydrocannabivarinsäure A (Δ9-THCVA-C₃ A), Δ9-Tetrahydrocannabiorcolsäure (Δ9-THCOA-C₁ A und/oder B), (-)-Δ8-trans-(6aR,10aR)-Δ8-Tetrahydrocannabinol (Δ8-THC-C₅),(-)-Δ8-trans-(6aR,10aR)-Tetrahydrocannabinolsäure A (Δ8-THCA-C₅ A); (-)-(6aS,10aR)-Δ9-Tetrahydrocannabinol ((-)-cis-Δ9-THC-C₅);
*Cannabinol-artige (CBN):* Cannabinol CBN-C₅, Cannabinol-C4 (CBN-C₄), Cannabivarin (CBN-C₃), Cannabinol-C2 (CBN-C₂), Cannabiorcol (CBN-C₁), Cannabinolsäure A (CBNA-C₅ A), Cannabinolmethylether (CBNM-C₅)
*Cannabitriol-artige (CBT) :* (-)-(9R,10R)-trans-Cannabitriol ((-)-trans-CBT-C₅), (+)-(9S,10S)-Cannabitriol ((+)-trans-CBT-C₅), (±)-(9R,10S/9S,10R)-Cannabitriol ((±)-cis-CBT-C₅), (-)-(9R,10R)-trans[10-0-Ethyl-cannabitriol] ((-)-trans-CBT-OEt-C₅), (±)-(9R,10R/9S,10S)-Cannabitriol-C3 ((±)-trans-CBT-C₃),8,9-Dihydroxy-Δ6a(10a) tetrahydrocannabinol (8,9-Di-OH-CBT-C₅), Cannabidiolsäure A (CBDA-C₅ 9-OH-CBT-C5 ester), (-)-(6aR,9S,10S,10aR)-9,10-Dihydroxy-hexahydrocannabinol, Cannabiripsol Cannabiripsol-C5, (-)-6a,7,10a-Trihydroxy-Δ9-tetrahydrocannabinol ((-)-Cannabitetrol), 10-Oxo-Δ6a(10a) tetrahydrocannabinol (OTHC);
*Cannabielsoin-artige (CBE) :* (5aS, 6S, 9R, 9aR) - C₅-Cannabielsoin (CBE-C₅), (5aS,6S,9R,9aR)-C₃-Cannabielsoin (CBE-C₃), (5aS,6S,9R,9aR)-Cannabielsoinsäure A (CBEA-C₅ A), (5aS,6S,9R,9aR)-Cannabielsoinsäure B (CBEA-C₅ B), (5aS,6S,9R,9aR)-C3-Cannabielsoinsäure B (CBEA-C₃ B), Cannabiglendol-C3 (OH-iso-HHCV-C₃), Dehydrocannabifuran (DCBF-C₅), Cannabifuran (CBF-C₅) ;
*Isocannabinoide:* (-)-Δ7-trans-(1R,3R,6R)-Isotetrahydrocannabinol, (±)-Δ7-1,2-cis-(1R,3R,6S/1S,3S,6R)-Isotetrahydrocannabivarin, (-)-Δ7-trans-(1R,3R,6R)-Isotetrahydrocannabivarin;
*Cannabicyclol-artige (CBL) :* (±)-(1aS,3aR,8bR,8cR-Cannabicyclol (CBL-C₅), (±)-(1aS,3aR,8bR,8cR-Cannabicyclolsäure A (CBLA-C₅ A), (±)-(1aS,3aR,8bR,8cR-Cannabicyclovarin (CBLV-C₃);
*Cannabicitran-artige (CBT) :* Cannabicitran (CBT-C₅) ;
*Cannabichromanon-artige (CBCN) :* Cannabichromanon (CBCN-C₅), Cannabichromanon-C3 (CBCN-C₃), Cannabicoumaronon (CBCON-C₅).

Neben den o. g. erwähnten Cannabinoiden finden sich deren zugehörige Carbonsäuren in der Rohdroge. Diese Carbonsäuren sind biosynthetische Precursors.

Cannabiszubereitungen üben eine Vielzahl therapeutischer Wirkungen aus, darunter antispastische, analgetische, antiemetische, neuroprotektive, antiinflammatorische sowie Wirkungen bei psychiatrischen Erkrankungen (Grotenhermen F, Müller-Vahl K: The therapeutic potential of cannabis and cannabinoids. Dtsch Arztebl Int 2012; 109(29-30): 495-501. DOI: 10.3238/arztebl.2012.0495).

In Deutschland ist seit 2011 ein Cannabisextrakt, der THC (Dronabinol) und CBD im Verhältnis 1:1 enthält (Nabiximols), für die Behandlung der mittelschweren bis schweren, therapieresistenten Spastik bei Multipler Sklerose (MS) als Sublingualspray (Sativex) arzneimittelrechtlich zugelassen.

Cannabidiol (CBD, CBD-C₅) ist das wichtigste nicht-psychotrope Cannabinoid der Gattung Cannabis und CBD ist kein Cannabinoidrezeptoragonist.

### Abb. 1: CBD (Strukturformel)

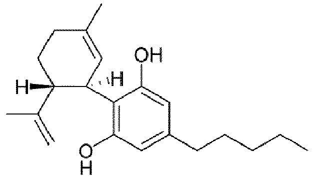

CBD kann synthetisch hergestellt werden (Michoulam R, Shvo Y., Hashish. I. The structure of cannabidiol, Tetrahedron. 1963, 19(12), 2073).

Weiterhin sind im Stand der Technik Verfahren zur Extraktion von Cannabidiol beschrieben:
DE 100 51 427 C1 (Müller) beschreibt einen CBD-haltigen Primärextrakt mittels CO₂-Extraktion aus Cannabis-Faserhanf unter Anwendung über- oder unterkritischem Druck und Temperaturbedingungen. Hierzu wird eine SFC- oder SFE-Anlage verwendet ("Superfluid-chromatography").

Eine geeignete Vakuumdestillation zur Extraktion von CBD in hoher Reinheit aus einem Cannabisrohmaterial wird jedoch im Stand der Technik nicht beschrieben. Im Stand der Technik wird die Vakuumdestillation von THC offenbart (WO 00/25127A1) und EP1051084B1 beschreibt z.B. eine Wasserdampfdestillaton aus Hanf.

Daher besteht die Aufgabe eine verbesserte Destillation zur Extraktion von CBD bereitzustellen, so dass möglichst ein THC-freier Extrakt, gar ein absolut THC-freier Extrakt enthaltend CBD bereitgestellt wird.

Die Aufgabe wird durch die angegebenen Patentansprüche gelöst.

Daher betrifft die Erfindung ein Verfahren zur Extraktion von Cannabidiol aus Cannabis-Pflanzenmaterial, wobei eine Vakuumdestillation durchgeführt wird (nachstehend: erfindungsgemäßes Verfahren).

Im Sinne dieser Erfindung bedeutet Destillation ein thermisches Trennverfahren, um verdampfbare Flüssigkeiten aus einer Gasphase zu gewinnen und von schwer verdampfbaren Stoffen abzutrennen, wobei ein Cannabidiol-haltiger Extrakt im Destillat angereichert wird. "Vakuumdestillation" im Sinne dieser Erfindung bedeutet, dass die Destillation im Vakuum bei 0,001 bis 50 mbar, vorzugsweise 0,001 bis 10 mbar, besonders bevorzugt 0,001 bis 1 mbar im so genannten Feinvakuumbereich erfolgt. Weiterhin ist bevorzugt, dass die Temperatur 120 bis 240 Grad Celsius beträgt, insbesondere 150 bis 230 Grad Celsius beträgt.

Besonders bevorzugt ist, dass das erfindungsgemäße Verfahren zur Vakuumdestillation mittels einer Kurzwegdestillation erfolgt. "Kurzwegdestillation" im Sinne dieser Erfindung bedeutet, dass die Gasphase im angelegten Feinvakuum nur einen sehr kurzen Weg zwischen der Vorlage und dem Kondensator zurückzulegen hat. Beispielsweise kann ein Kurzwegverdampfer verwendet werden, der konstruktiv einem konventionellen Dünnschichtverdampfer entspricht, wobei jedoch der Kondensator in das Innere des Verdampferzylinders integriert ist, so dass der Weg den die Dämpfe bis zum Kondensator zurücklegen müssen, sehr kurz ist und Drücke von 0,001 mbar erreicht werden können.

Erfindungsgemäß umfasst ist ebenfalls ein erfindungsgemäßes Verfahren mittels eines Dünnschichtverdampfers oder Fallfilmverdampfers. Dünnschichtverdampfer und Fallfilmverdampfer sind an sich bekannte thermische Trennapparate.

Ein geeigneter Dünnschichtverdampfer (engl.: thin film evaporator) im Sinne dieser Erfindung weist eine im Wesentlichen zylindrische, mit Dampf beheizte Innenwandung auf, auf der mittels rotierender Verteilorgane eine dünne Schicht eines Primärextrakts aufgebracht wird. Die motorisch angetriebenen Verteilorgane werden benötigt, um das rasch auf den Platten verdampfende Gemisch auszubringen und zu verteilen.

Ein geeigneter Fallfilmverdampfer (engl.: falling film evaporator) im Sinne dieser Erfindung umfasst ein im Wesentlichen vertikal verlaufendes, von außen beheiztes Rohr, an dessen Innenseite ein Primärextrakt in einem dünnen Film herab rinnt und dabei verdampft. Die unverdampften Komponenten werden am bodenseitigen Ende des Rohrs als Sumpf abgezogen, die verdampften Komponenten verlassen das andere Ende des Rohrs als Kopfprodukt. Der Fallfilmverdampfer kommt mithin ohne bewegliche Teile aus. Gemeinsames konstruktives Merkmal von Dünnschichtverdampfer und Fallfilmverdampfer ist zumindest ein beheiztes, flächiges Verdampfungsorgan, auf dem eine dünne Schicht des flüssigen Primärextrakts aufgegeben und teilweise verdampft wird (ULLMANN: Billet, Reinhard: Evaporation, Ullmann's Encyclopedia of Industrial Chemistry, Published Online: 15 JUN 2000 DOI: 10.1002/14356007.b03_03, VTA Verfahrenstechnische Anlagen GmbH & Co. KG, Niederwinkling (DE)).

Erfindungsgemäß bevorzugt ist jedoch ein Kurzwegverdampfer oder ein Dünnschichtverdampfer.

In einer weiteren Ausführungsform der Erfindung kann die Vakuumdestillation mit einer Kolonnendestillation gekoppelt werden. Insbesondere kann ein Kurzwegverdampfer, Dünnschichtverdampfer oder Fallfilmverdampfer mit einer Kolonne entsprechend bestückt werden. Im Sinne dieser Erfindung ist eine Kolonnendestillation ebenfalls eine geeignete Rektifikation im Vakuum, auch unter Rückfluss, welche vorzugsweise 10 Trennstufen aufweist. Die Rektifikation erlaubt die sichere Abtrennung von THC und Anreicherung von CBD auf mehr als 80 %.

Bevorzugt ist weiterhin, dass die erfindungsgemäße Vakuumdestillation mit einer gekoppelten Kolonnendestillation bei einem Druck von 0,001 bis 50 mbar, vorzugsweise 0,001 bis 10 mbar, insbesondere 0,001 bis 1 mbar erfolgt. Weiterhin ist bevorzugt, dass die Temperatur 120 bis 240 Grad Celsius beträgt, insbesondere 150 bis 230 Grad Celsius beträgt. In einer bevorzugten Ausführungsform beträgt der Druck bis 5 - 10 mbar und die Temperatur 200 bis 230 Grad Celsius. Dies erlaubt die sichere CBD Anreicherung unter Erhalt eines THC-freien Extrakts, wobei THC vollständig abgereichert wird.

Weiterhin ist bevorzugt, dass die Vakuumdestillation an einem ersten Primärextrakt mit mindestens 15 Gew. % an CBD erfolgt. In einem ersten Schritt wird daher das Cannabis-Pflanzenmaterial geschnitten, zerkleinert und einer ersten Extraktion unterzogen, beispielsweise eine CO₂ Extraktion, wie in DE 100 51 427 C1 beschrieben. Ebenfalls kann alternativ eine Extraktion mit Hexan oder eine Säulenchromatographie erfolgen und auf diese Weise ein Primärextrakt vorzugsweise mit mindestens 15 Gew. % an CBD erhalten werden, welcher zur weiteren Vakuumdestillation eingesetzt wird (supra). Eine CO₂-Extraktion ist jedoch bevorzugt. Bevorzugtes Cannabis-Pflanzenmaterial ist Faser-, Nutzhanf, insbesondere der Sorten Fedora 17, Felina 34, Ferimon 12, Futura 75 u.a. mit relativ hohem Gehalt an CBD in Gew. %.

Daher ist Gegenstand der Erfindung ebenfalls ein Cannabidiol-haltiger Extrakt erhältlich nach einem erfindungsgemäßen Verfahren. Der Extrakt ist honigfarben, streichfähig und von angenehmem Geruch und weist einen CBD-Gehalt von mehr als 35 Gew. %, insbesondere mehr als 50 Gew. % im Trockengewicht auf und ist nahezu THC-frei oder gar absolut THC-frei.

Besonders vorteilhaft kann dieser Cannabidiol-haltige Extrakt aufgrund seiner hervorragenden Konsistenz in Formulierungen wie Salbe, Creme, Gel, Lotion (Hautmilch), Paste oder vorzugsweise Emulsion sofort eingesetzt werden.

Daher betrifft die Erfindung ebenfalls ein Arzneimittel, Nahrungsergänzungsmittel oder Kosmetikum enthaltend einen Cannabidiol-haltigen Extrakt erhältlich nach einem erfindungsgemäßen Verfahren. Das Arzneimittel ist für die antispastische, analgetische, antiemetische, neuroprotektive, antiinflammatorische Wirkung sowie bei psychiatrischen Erkrankungen geeignet.

Die galenische Herrichtung einer erfindungsgemäßen pharmazeutischen Zusammensetzung oder Mittel, insbesondere ein Arzneimittel (Medikament), kann in fachüblicher Weise erfolgen. Geeignete feste oder flüssige galenische Zubereitungsformen sind beispielsweise Granulate, Pulver, Dragees, Tabletten, (Mikro-) Kapseln, Hartkapseln, Suppositorien, Sirupe, Säfte, Suspensionen, oder Emulsionen, bei deren Herstellung übliche Hilfsmittel wie Trägerstoffe, Spreng-, Binde-, Überzugs-, Quellungs-, Gleit™ oder Schmiermittel, Geschmacksstoffe, Süßungsmittel und Lösungsvermittler, Verwendung finden. Als Hilfsstoffe seien Magnesium-Stearat, Natriumchlorid, Magnesiumcarbonat, Titandioxid, Lactose, Mannit und andere Zucker, Talcum, Milcheiweiß, Gelatine, Stärke, Zellulose und ihre Derivate, tierische und pflanzliche Öle wie Lebertran, Sonnenblumen-, Erdnuss- oder Sesamöl, Polyethylenglycole und Lösungsmittel, wie etwa steriles Wasser und ein- oder mehrwertige Alkohole, beispielsweise Glycerin, genannt.

Weiterhin können als Hilfs- und Zusatzstoffe weitere Detergentien und Tenside vorgesehen sein, wie beispielsweise nachstehend für eine kosmetische Zusammensetzung ausgeführt.

Unter Emulsionen, insbesondere für eine kosmetische Zusammensetzung versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert. Sind die beiden Flüssigkeiten Wasser und Öl und liegen Öltröpfchen fein verteilt in Wasser vor, so handelt es sich um eine Öl-in-Wasser-Emulsion (O/W-Emulsion). Der Grundcharakter einer O/W-Emulsion ist durch das Wasser geprägt. Bei einer Wasser-in-Öl-Emulsion (W/O-Emulsion) handelt es sich um das umgekehrte Prinzip, in dem der Grundcharakter hier durch das Öl bestimmt wird. Weiterhin sind Mischsysteme wie Wasser-in-Öl-in-Wasser-Emulsionen (W/O/W-Emulsion) und Öl-in-Wasser-in-Öl-Emulsionen (O/W/O-Emulsionen) bekannt. Alle genannten Emulsionen sind erfindungsgemäß geeignet.

Zu den erfindungsgemäß geeigneten wasserfreien Systemen gehören reine Ölzubereitungen wie z.B. Hautöle. Ebenfalls einsetzbare Pasten enthaltend die erfindungsgemäße Zubereitung und zeichnen sich dadurch aus, das sie aus den gleichen oder ähnlichen Bestandteilen wie eine Emulsion bestehen, jedoch im Wesentlichen wasserfrei sind. Im Rahmen der vorliegenden Erfindung werden die Begriffe Ölphase und Lipidphase synonym verwendet. In einer weiteren bevorzugten Ausführungsform kann die erfindungsgemäße Zubereitung als weiteren Bestandteil einen Emulgator beinhalten. In einer ganz bevorzugten Ausführungsform kann dieser Emulgator ein O/W-Emulgator sein. Emulgatoren können vorteilhaft gewählt werden aus der Gruppe der nichtionischen, anionischen, kationischen oder amphoteren Emulgatoren.

Als nichtionischen Emulgator können verschiedene Emulgatoren aus den Gruppen der Partialfettsäureester, Fettalkohole, Sterole, Polyethylengylcole wie z.B. ethoxylierte Fettsäuren, ethoxylierte Fettalkohole und ethoxylierte Sorbitanester, Zuckeremulgatoren, Polyglycerinemulgatoren oder Silikonemulgatoren Verwendung finden.

Als anionischen Emulgator können verschiedene Emulgatoren aus den Gruppen der Seifen z. B. Natriumstearat, Fettalkoholsulfate, Mono-, Di- und Trialkylphosphosäureester und deren Ethoxylate, Fettsäure Lactat Ester, Fettsäure Citrat Ester oder Fettsäure Citroglycerinester Verwendung finden.

Als kationischen Emulgatoren können beispielsweise quaternäre Ammoniumverbindungen mit einem langkettigen aliphatischen Rest z. B. Distearyldimonium Chloride eingesetzt werden.

Unter den amphoteren Emulgatoren können verschiedene Emulgatoren aus den Gruppen Alkylamininoalkancarbonsäuren, Betaine, Sulfobetaine oder Imidazolinderivate Verwendung finden.

Erfindungsgemäß bevorzugt sind natürlich vorkommende Emulgatoren, zu denen beispielsweise Bienenwachs, Wollwachs, Lecithin und Sterole u.a. gehören die ebenfalls bei der Herstellung einer erfindungsgemäßen Zubereitung eingesetzt werden können. In einer bevorzugten Formulierung der erfindungsgemäßen Zubereitung können O/W-Emulgatoren ausgewählt werden aus der Gruppe der Pflanzenproteinhydrolysate und deren Derivate.

Im Sinne der vorliegenden Erfindung können ferner vorteilhaft Substanzen als Zusatzstoffe enthalten sein, ausgewählt aus der Gruppe der Ester aus gesättigten und/oder ungesättigten, verzweigten und/oder unverzweigten Alkancarbonsäuren und/oder Alkencarbonsäuren mit einer Kettenlänge von 3 - 30 Kohlenstoffatomen und gesättigten und/oder ungesättigten, verzweigten und/oder unverzweigten Alkoholen einer Kettenlänge von 3 - 30 Kohlenstoffatomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esterole können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononyliso-nonanoat, 2-Ethylhexylpalmitat, 2-Ethylhexyllaurat, 2- Hexyldecylstearat, 2-Octyldodecyl-palmitat, Oleyloleat, Oleylerucat, Erucyloleat, Erucylerucat sowie synthetische, halbsynthetische und natürliche Gemische solcher Ester, wie z.B. Jojobaöl.

Ferner kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 - 24 C-Atomen, insbesondere 12 - 18 C-Atomen. Die Fettsäuretriglyceride können beispielsweise vorteilhaft gewählt werden aus der Gruppe der synthetischen, halbsynthetischen und natürlichen Öle.

Insbesondere können in den erfindungsgemäßen Zubereitungen zusätzlich als Hilfs- oder Zusatzstoff Antioxidantien und/oder Radikalfänger zugesetzt werden. Vorteilhaft werden solche Antioxidantien gewählt aus der Gruppe der lipophilen System beispielsweise: natürliche und synthetische Tocopherole, Nordihydroguajaretsäure, Coniferylbenzoat, Butylhydroxyanisol, Butlyhydroxy-tolul, Gallussäureester und verschiedene antioxidative Pflanzen Extrakte. Unter den hydrophilen Systemen sind besonders vorteilhaft anorganischen Schwefelverbindungen, Natriumhydrogensulfit, Cystein oder Ascorbinsäure zu verwenden.

Die erfindungsgemäßen kosmetischen Zubereitungen können weiterhin kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, weichmachende, anfeuchtende und/oder feuchthaltende Substanzen oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

In einer weiteren besonderen Ausführungsform ist die erfindungsgemäße Zubereitung im Wesentlichen aus natürlich vorkommenden Inhaltsstoffen zusammengesetzt, wie oben genannt.

## Patentansprüche

1. Verfahren zur Extraktion von Cannabidiol aus Cannabis-Pflanzenmaterial, **dadurch gekennzeichnet, dass** eine Vakuumdestillation durchgeführt wird.

2. Verfahren zur Extraktion von Cannabidiol aus Cannabis-Pflanzenmaterial nach Anspruch 1, **dadurch gekennzeichnet, dass** die Vakuumdestillation eine Kurzwegdestillation ist.

3. Verfahren zur Extraktion von Cannabidiol aus Cannabis-Pflanzenmaterial nach Anspruch 1 oder Anspruch 2, **dadurch gekennzeichnet, dass** die Vakuumdestillation mittels einem Dünnschichtverdampfer, Kurzwegverdampfer oder Fallfilmverdampfer erfolgt.

4. Verfahren zur Extraktion von Cannabidiol aus Cannabis-Pflanzenmaterial nach einem der vorstehenden Ansprüche, wobei die Vakuumdestillation an einem ersten Primärextrakt mit mindestens 15 Gew. % an CBD erfolgt.

5. Verfahren zur Extraktion von Cannabidiol aus Cannabis-Pflanzenmaterial nach einem der vorstehenden Ansprüche, wobei der Druck 0,001 bis 50 mbar und die Temperatur 120 bis 240 Grad Celsius beträgt.

6. Verfahren zur Extraktion von Cannabidiol aus Cannabis-Pflanzenmaterial nach einem der vorstehenden Ansprüche, wobei die Vakuumdestillation mit einer Kolonnendestillation gekoppelt ist.

7. Cannabidiol-haltiger Extrakt erhältlich nach einem Verfahren gemäß den Ansprüchen 1 bis 6.

8. Arzneimittel, Nahrungsergänzungsmittel enthaltend einen Cannabidiol-haltigen Extrakt erhältlich nach einem Verfahren gemäß den Ansprüchen 1 bis 6.

9. Kosmetikum enthaltend einen Cannabidiol-haltigen Extrakt erhältlich nach einem Verfahren gemäß den Ansprüchen 1 bis 6.

10. Verwendung eines Cannabidiol-haltigen Extrakts nach Anspruch 7 als Kosmetikum oder Nahrungsergänzungsmittel.
